# EUROPÄISCHE PATENTANMELDUNG

(11) **EP 1 331 482 A1**
(43) Veröffentlichungstag der Anmeldung: **30.07.2003**
(21) Anmeldenummer: 02001808.1
(22) Anmeldetag: 25.01.2002
(51) Int. Cl.: G01N 33/531

(54) **Konjugat zur Bestimmung von Antigenen mittels Biosensoren**

(71) Anmelder: BMS Sensor Technology SA, 2001 Neuchatel (CH)
(72) Erfinder: Mattman, Thomas, 3072 Ostermundingen (CH); Hug, Werner, 3308 Grafenried (CH); Schibli, Peter, 4503 Solothurn (CH)
(74) Vertreter: Störle, Christian, Dr.

(57) **Zusammenfassung**

Es wird ein Konjugat zur Bestimmung von Antigenen mittels Biosensoren beschrieben, das einen Mediator und ein Antigen, die kovalent miteinander verbunden sind, sowie einen gegen das Antigen gerichteten Antikörper, der über eine Antigen-Antikörper-Bindung gebunden ist, umfaßt. Ferner wird ein Biosensor, der das Konjugat aufweist, sowie ein Verfahren zur Bestimmung von Antigenen unter Verwendung des Konjugats beschrieben.

## Beschreibung

Die Erfindung bezieht sich auf ein Konjugat zur Bestimmung von Antigenen mittels Biosensoren, ein Verfahren zur Bestimmung von Antigenen und einen dieses Konjugat aufweisenden Biosensor.

In der jüngsten Zeit werden vermehrt Biosensoren zum Nachweis von Substanzen (Analyt) eingesetzt. Biosensoren stellen eine Kopplung biologischer Komponenten zur spezifischen Erkennung des Analyten mit einem physikalischen Transduktor (Signalwandler) dar. Sie wandeln die biochemischen Informationen in ein physikalisch kondifizierbares Signal um, beispielsweise in ein elektrisches Signal, das einer elektronischen Verstärkung zugänglich ist. Dabei kommen amperometrische Elektroden als Transduktoren zum Einsatz (Römpp-Lexikon Biotechnologie, Gentechnik, Stuttgart, New York, 1999, S. 120).

In solchen Biosensoren werden oftmals sogenannte Mediatoren eingesetzt. Darunter versteht man niedermolekulare Redoxüberträger, die den Elektronentransfer, z. B. von einem Enzym zur Elektrode, vermitteln (a.a.O., S. 494).

Beispielsweise kann mittels eines Biosensors Glucose im Harn oder im Blut unter Verwendung des Enzyms Glucoseoxidase bestimmt werden. Dazu wird die Glucose durch die Glucoseoxidase zu Gluconolacton oxidiert. Die dabei frei werdenden Elektronen reduzieren die Glucoseoxidase, das dann wiederum Elektronen auf einen Mediator, beispielsweise Ferrocen, überträgt, wobei das Enzym seinerseits oxidiert wird. Vom Mediator können dann die Elektronen auf eine Elektrode übertragen werden, so daß bei Anlegen einer Spannung ein Mikrostrom fließt.

Diese enzymatische Reaktion kann in einem Biosensor elektrochemisch erfaßt werden, indem nach Anlegen der Spannung ein Strom zwischen zwei Elektroden gemessen wird (amperometrische Messung). Der Strom dient als Maß für den Glucosegehalt der Blutprobe.

Biosensoren, die zur Erkennung des Analyten Antikörper besitzen, werden als Immunosensoren bezeichnet. Diese nutzen die spezifische Reaktion von Antikörpern mit Antigenen. Die bisher bekannten Immunosensoren weisen jedoch ganz erhebliche Nachteile auf, die deren Verwendung stark einschränken. So sind sie im allgemeinen nicht tragbar und können deshalb nur an einem vorbestimmten Ort, nicht jedoch dort, wo sie gerade benötigt werden, eingesetzt werden. Zur Durchführung und Auswertung sind teure und aufwendige Geräte erforderlich. Die Immunosensoren sind insgesamt sehr teuer.

Der vorliegenden Erfindung liegt somit die Aufgabe zugrunde, ein Mittel bereitzustellen, das die Messung von Antigenen (Analyten) mittels Biosensoren, insbesondere Immunosensoren, ermöglicht, ohne daß dabei die vorstehend geschilderten Nachteile auftreten.

Erfindungsgemäß wird dies erreicht durch ein Konjugat zur Bestimmung von Antigenen mittels Biosensoren, das einen Mediator und ein Antigen, die kovalent miteinander verbunden sind, sowie einen gegen das Antigen gerichteten Antikörper, der über eine Antigen-Antikörper-Bindung gebunden ist, umfaßt.

Der Ausdruck Konjugat weist darauf hin, daß dessen Bestandteile Mediator, Antigen und Antikörper über gerichtete Bindungen in einer definierten Anordnung vorliegen. So ist der Mediator an das Antigen kovalent gebunden. Solche kovalenten Bindungen können beispielsweise Esteroder Amid-Bindungen sein. Diese können z. B. dadurch gebildet werden, daß eine Carboxyl-Gruppe oder aktivierte Carboxyl-Gruppe des Mediators mit einer Hydroxyl- oder Amino-Gruppe des Antigens oder eine Carboxyl-Gruppe oder aktivierte Carboxyl-Gruppe des Antigens mit einer Hydroxyl- oder Amino-Gruppe des Mediators umgesetzt wird. Da der im erfindungsgemäßen Konjugat vorliegende Antikörper gegen das Antigen gerichtet ist und somit gegen das Antigen spezifisch ist, entsteht ferner eine definierte Bindung zwischen Antigen und Antikörper.

In einer bevorzugten Ausführungsform des erfindungsgemäßen Konjugats ist der Mediator ein Ladungstransferkomplex, der Donor und Akzeptor aufweist, oder ein Redoxmediator. Diese, insbesondere in den nachfolgend geschilderten bevorzugten Ausführungsformen, sind günstig, da sie in besonders vorteilhafter Weise als Elektronenakzeptoren für Oxidoreduktasen wirken. Ferner sind sie zur Übertragung von Elektronen auf Elektroden bestens geeignet.

Aufgrund ihrer vorteilhaften Eigenschaften hinsichtlich der Elektronenübertragung umfassen der Redoxmediator günstigerweise Ferrocen, Nickelocen oder ein Derivat von diesen, wie vorzugsweise Ferrocen-aminopropylpyrrol, Ferrocen-aminopentylamidopropylpyrrol und Ferrocen-1,1'-C₁-C₁₀-Dicarbonsäuren, insbesondere Ferrocen-1,1'-Dimethylbutansäure.

Bei Ladungstransferkomplexen handelt es sich um Verbindungen, die durch den partiellen Übergang eines Elektrons von einem Donor auf einen Akzeptor stabile Komplexe bilden und in der Lage sind, Ladungen zu transferieren. Vorzugsweise ist der Donor ausgewählt unter Tetrathiofulvalen (TTF), Hexamethylentetraselenfulvalen (HMTSF), Tetramethyltetraselenfulvalen (TMTSF), N-Methylphenazon (NMP), Methyl-norbornen-2,3-dicarbonsäureanhydrid (NMA), Chinolin (Q), Kupfer-2,2-Dipyridylamin (DPA) und 2,5-Dimethoxyzimtsäure. Die Akzeptoren sind vorzugsweise ausgewählt unter 7,7,8,8-Tetracyanochinodimethan (TCNQ), 7,7,8,8-Tetracyanochinodimethantetrafluorid (TCNQF₄), 2-[(3,5-Dinitro-phenyl)-(3-nitrophenyl)-methylen]-molononitril (DTF), 11,11,12,12-Teracyan-naphtochinodimethan-(2,6) (TNAP) und Phenazinmethosulfat. Auch Salze der vorstehenden Donoren und/oder Akzeptoren können im erfindungsgemäßen Konjugat eingesetzt werden.

Besonders bevorzugte Vertreter der Ladungstransferkomplexe sind ausgewählt unter Tetrathiofulvalen-7,7,8,8-Tetracyanoquinodimethan (TTF⁺-TCNQ⁻), Mehyl-norbornen-2,3-dicarbonsäure-anhydrid-7,7,8,8-tetracyanoquinodimethan (NMA⁺-TCNQ⁻) oder 1-Methyl-2-pyrrolidon-7,7,8,8-Tetracyanoquinodimethan (NMP⁺-TCNQ⁻).

Wie bereits vorstehend ausgeführt wurde, ist im erfindungsgemäßen Konjugat an vorgenanntem Mediator kovalent ein Antigen gebunden. Unter Antigen wird dabei jede Substanz verstanden, gegen die Antikörper gebildet werden können. Bei dem im erfindungsgemäßen Konjugat vorliegenden Antigen handelt es sich um die Substanz (Analyt), die mit dem Biosensor nachgewiesen werden soll. Als Antigene können körpereigene oder körperfremde Substanzen jeglicher Art eingesetzt werden. Es kann sich beispielsweise um Krankheitserreger, wie Viren und Bakterien, oder Bestandteile davon, biologisch relevante Moleküle jeglicher Art, wie Proteine, Peptide, Glycoproteine, Lipoproteine oder Nucleinsäuren, handeln. Bei Peptiden handelt es sich um aus Aminosäuren aufgebaute Verbindungen mit einem Molekulargewicht von weniger als etwa 1000; Proteine weisen ein Molekulargewicht von mehr als 1000 auf.

Im erfindungsgemäßen Konjugat liegt, wie bereits vorstehend ausgeführt wurde, ein gegen das Antigen gerichteter Antikörper vor, der über eine Antigen-Antikörper-Bindung gebunden ist. Der Antikörper kann ein monoklonaler oder ein polyklonaler Antikörper sein. Der Fachmann kennt Verfahren und hierzu notwendige Materialien, um für das Antigen spezifische Antikörper herzustellen. Das Verhältnis von Antikörper zu der Verbindung aus Antigen und Mediator kann dabei so gewählt werden, daß nicht alle Bindungsstellen des Antikörpers mit dem Antigen belegt sind. Beispielsweise sind 1 % - 15 %, insbesondere etwa 10 %, der Bindungsstellen des Antikörpers für das Antigen nicht damit besetzt. Dies kann dadurch erreicht werden, daß ein Unterschuß der Verbindung aus Antigen und Mediator mit dem Antikörper umgesetzt wird. Durch dieses vorgenannte Verhältnis wird erreicht, daß kein nicht an den Antikörper gebundener Mediator mehr vorliegt und auf die Elektroden im Biosensor aufgebracht wird. Dadurch wird bewirkt, daß ein Nullstrom (Rauschen) im Sensor vermieden wird, das durch eine Elektronenübertragungsreaktion der ungebundenen Verbindung aus Antigen und Mediator erzeugt wird.

Das erfindungsgemäße Konjugat kann in besonders leichter, einfacher, kostengünstiger und schneller Weise hergestellt werden. Dazu kann das Antigen in üblicher Weise mit dem Mediator kovalent verbunden werden, beispielsweise über eine Ester- oder Amid-Bindung, wie sie vorstehend erläutert wurde.

Zur Veranschaulichung ist die Bindung von Ferrocencarbonsäure, wobei der Ferrocen-Bestandteil als Mediator fungiert, an humanes Serumalbumin (HSA) als Antigen im nachfolgenden Formelschema dargestellt.

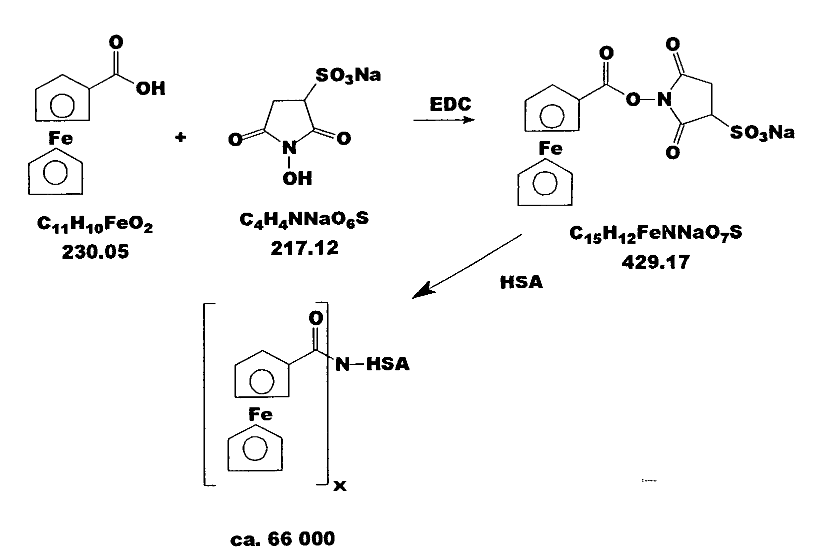

Das erfindungsgemäße Konjugat, insbesondere in den vorgenannten bevorzugten Ausführungsformen, weist eine Vielzahl von günstigen Eigenschaften auf, so daß es in einem Biosensor zum besonders günstigen Nachweis des Antigens eingesetzt werden kann.

So kann das erfindungsgemäße Konjugat kostengünstig, einfach und schnell hergestellt werden. Bei der Herstellung kann es in hoher Reinheit erhalten werden. Die erfindungsgemäßen Konjugate sind gut definiert, wodurch unerwünschte Nebenreaktionen bei der Bestimmung des Antigens durch Verunreinigungen nahezu ausgeschlossen sind. Des weiteren kann durch die geeignete Wahl des Mediators in Bezug auf das einzusetzende Antigen und durch geeignete Wahl von ggf. am Mediator vorliegenden Substituenten dessen Fähigkeit zur Elektronenübertragung so günstig beeinfluß werden, daß optimale Meßergebnisse mit einem Biosensor erhalten werden können. Auf diese Weise ist eine sehr gute Feinabstimmung auf den nachzuweisenden Analyten und die Art der Probe, in der er vorliegt, möglich.

Das erfindungsgemäße Konjugat ist aufgrund vorstehender Eigenschaften bestens zum Nachweis des Antigens in Proben, beispielsweise Körperflüssigkeiten, wie Blut und Speichel, mittels eines Biosensors geeignet. Dabei können insbesondere an sich bekannte Biosensoren mit amperometrischen Elektroden eingesetzt werden, wie sie beispielsweise in der EP 1 167 538 A beschrieben sind, auf die hiermit ausdrücklich Bezug genommen wird. Bei solchen amperometrischen Elektroden wird nach Anlegen einer Spannung ein Strom zwischen zwei Elektroden gemessen, vgl. vorstehende Ausführungen.

Bei einem erfindungsgemäßen Verfahren zum Nachweis des Antigens mit einem Biosensor, der a) Elektroden, die eine elektrochemische, insbesondere amperometrische Messung, ermöglichen, b) das erfindungsgemäße Konjugat c) eine Oxidoreduktase mit Sauerstoff als Akzeptor, insbesondere Glucoseoxidase, und d) ein Substrat für diese Oxidoreduktase, z. B. Glucose, aufweist, wird dieser Biosensor mit der zu untersuchenden Probe in Kontakt gebracht und eine elektrochemische, insbesondere amperometrische Messung durchgeführt.

Wie vorstehend ausgeführt wurde, weist der Biosensor das erfindungsgemäße Konjugat zusammen mit der Oxidoreduktase und dem Substrat dafür auf. Beispiele für Oxidoreduktasen und Substrat sind Glucoseoxidase und Glucose, die besonders geeignet sind, da die Elektronenübertragung in Biosensoren besonders gut abläuft.

Dabei kann die Elektrode mit dem erfindungsgemäßen Konjugat, der Oxidoreductase und dem Substrat beschichtet sein. Eine solche Beschichtung kann beispielsweise mit einem Siebdruckverfahren erfolgen. Dabei können folgende Siebdruckkomponente eingesetzt werden: 5 Gew.% bis 25 Gew.% erfindungsgemäßes Konjugat, 10 Gew.% bis 30 Gew.% Oxidoreduktase, 0,5 Gew.% bis 5 Gew.% Substrat für die Oxidoreduktase, wobei der Rest zu 100 Gew.% eine Trägersubstanz ist, insbesondere 5 Gew.% bis 25 Gew.% Graphit und 40 Gew.% bis 60 Gew.% Silikonöl. Die Herstellung des Gemischs der Siebdruckkomponenten kann dadurch erfolgen, daß Antikörper und die Verbindung von Antigen und Mediator und die Oxidoreduktase zusammen lyophilisiert werden, die anderen Komponenten dazugemischt werden, mehrmals mit Cyclohexanol versetzt und im Hochvakuum getrocknet wird.

Die Wirkungsweise des erfindungsgemäßen Konjugats im vorbeschriebenen Nachweisverfahren wird nachfolgend erläutert, ohne jedoch daran gebunden zu sein.

Wie bereits einleitend ausgeführt wurde, wird Glucose durch Glucoseoxidase zu Gluconolacton oxidiert. Die dabei frei werdenden Elektronen werden letztendlich auf den Mediator und von diesem auf eine Elektrode übertragen. Diese enzymatische Reaktion kann in einem Biosensor elektrochemisch erfaßt werden, z. B. in dem nach Anlegen einer Spannung ein Strom zwischen zwei Elektroden gemessen wird.

Es wurde nun überraschenderweise gefunden, daß im erfindungsgemäßen Konjugat, diese Elektronenübertragung des Mediators inhibiert ist. Dies bedeutet, daß kein Strom fließt. Wird aber nun der Antikörper aus dem erfindungsgemäßen Konjugat verdrängt, z. B. durch weiteres nachzuweisendes Antigen, das mit dem Antigen-Mediator um die Bindung an den Antikörper konkurriert, so kann die nun freigewordene Verbindung von Antigen und Mediator Elektronen übertragen. Der dabei fließende Strom ist direkt proportional zur Menge des nachzuweisenden Antigens, wodurch sowohl eine qualitative als auch quantitative Bestimmung des Antigens ermöglicht wird.

Bei dem erfindungsgemäßen Verfahren fungiert die Oxidoreduktase und das Substrat als Indikatorreaktion, die die Elektronenübertragung ermöglicht; nachgewiesen wird jedoch das Antigen.

Das erfindungsgemäße Verfahren weist eine Vielzahl von Vorteilen auf. Art und Konzentration des Antigens können mit hoher Spezifität und hoher Empfindlichkeit bestimmt werden. Naßchemische Schritte, wie sie ansonsten bei Immunoassays notwendig sind, insbesondere das Waschen (Trennen von gebundenen und ungebundenen Antigenen) ist nicht notwendig. Auch fallen beim Durchführen des erfindungsgemäßen Verfahrens keine toxischen Abfälle an, die möglicherweise unter Einhaltung gesetzlicher Bestimmungen aufwendig entsorgt werden müssen. Dadurch, daß das erfindungsgemäße Verfahren mittels Biosensoren durchgeführt werden kann, wie sie an sich beispielsweise zur Blutzuckermessung bekannt sind, ist eine Miniaturisierung möglich. Die zur Durchführung des erfindungsgemäßen Verfahrens notwendigen Geräte können deshalb so ausgestaltet sein, daß sie ohne Probleme transportiert werden können, wodurch der Einsatz vor Ort möglich ist. Zur Durchführung des erfindungsgemäßen Verfahrens sowie zur Auswertung der dadurch erhaltenen Ergebnisse sind keine aufwendigen Laboratorien notwendig, zu denen die Proben zuerst geschickt werden müssen. Die Analyseergebnisse stehen sehr schnell und in einfacher Weise zur Verfügung. Da keine komplizierten und teuren Geräte zur Durchführung des erfindungsgemäßen Verfahrens benötigt werden, ist das Verfahren sehr kostengünstig.

Gegenstand der vorliegenden Erfindung ist ferner ein Biosensor zum Bestimmen von Substanzen, wobei Elektroden vorgesehen sind, die zusammen mit einer enzymhaltigen Substanz eine elektrochemische Messung der Substanz erlauben, wobei der Sensor ein erfindungsgemäßes Konjugat aufweist. Für den erfindungsgemäßen Biosensor können an sich bekannte Biosensoren eingesetzt werden. Besonders geeignet ist der Biosensor, der in der EP 1 167 538 beschrieben ist, auf die hiermit ausdrücklich insbesondere hinsichtlich der konstruktiven Details des Sensors Bezug genommen wird. Dabei kann die Elektrode mit dem erfindungsgemäßen Konjugat und den weiteren Bestandteilen für die Messung (Oxidoreduktase und Substrat dafür) beschichtet sein, z. B. mit vorstehenden Siebdruckkomponenten.

Die nachfolgenden Beispiele sollen die Erfindung erläutern, ohne sie jedoch daraufhin einzuschränken.

### Beispiel:

### 1. Herstellung des erfindungsgemäßen Konjugats

In einem ersten Schritt erfolgt die kovalente Anbindung von Ferrocen an humanes Serumalbumin (HSA) (vgl. vorstehendes Formelschema).

Dazu wurden 3,54 g Ferrocencarbonsäure in 30 ml DMF gelöst. Zu dieser Lösung wurden 3,84 g EDC-chlorid und 4,34 g N-Hydroxysulfosuccinimid Natriumsalz in 30 ml DMF und 70 ml H₂O während 10 Minuten zugetropft. Das Reaktionsgemisch wurde dann während 24 Stunden bei Raumtemperatur gerührt. Es wurde eine aktivierte Ferrocencarbonsäure (Aktivester) erhalten.

Zu Humanem Serumalbumin (5 g), das in 100 ml Phosphat-gepufferter Salzlösung und 200 ml Wasser gelöst war, wurden 15 ml der vorbezeichneten Lösung des Aktivesters während 10 Minuten zugetropft. Das Reaktionsgemisch wurde dann während 4 Stunden bei Raumtemperatur gerührt. Die gerührte Lösung wurde 3 Tage im Kühlschrank bei 4°C gelagert. Anschließend wurde über einen 0,45 µm Membranfilter filtriert und danach das Produkt in der Bioableitung ultrafiltriert. Es wurde eine Verbindung aus dem Mediator Ferrocen und dem Antigen HSA erhalten, die über eine Esterbindung kovalent miteinander verbunden sind. Die Produktidentifizierung erfolgte über UV-Spektroskopie.

Für die Umsetzung dieser Verbindung mit dem Antikörper wurde ein monoklonaler Antikörper eingesetzt (Monodonal Anti-Human Serum Albumin, Clone HSA-11 von Mouse Ascites Fluid, Sigma A6684). Nach dem Analysezertifikat hatte das Produkt folgende Eigenschaften: Protein nach Biuret 35 mg/ml, IGG2A durch RID 9 mg/ml. Der eigentliche Antikörper ist also mit 9 mg/ml anzunehmen. Der Antikörper weist zwei Bindungsstellen auf, so daß ein 1:2 Komplex (Antikörper zu vorstehender Verbindung) zur Absättigung des Konjugats führt. Der Antikörper hat eine Masse von ca. 175 kD, die vorgenannte Verbindung von etwa 66 kD. Daraus ergibt sich ein Masseverhältnis von Antikörper zu vorgenannter Verbindung von 175:2 x 66. Dies entspricht 6,8 mg vorgenannter Verbindung aus Ferrocen und HSA zur vollständigen Belegung der Bindungsstellen des Antikörpers. Eingesetzt wurden 6 mg dieser Verbindung, wodurch sich ein mehr als 10%iger Überschuß von noch freien Antikörperbindungsstellen ergibt. Auf diese Weise ist sichergestellt, daß die Verbindung aus Ferrocen und HSA quantitativ an den Antikörper gebunden ist; es liegt keine freie Verbindung mehr vor. Dadurch wird erreicht, daß ein durch diese freie Verbindung im Biosensor erzeugter Nullstrom (= Rauschen) vermieden wird und somit bei der Messung mit einem Biosensor ein sehr günstiges Signal:Rausch-Verhältnis erhalten wird.

### 2. Beschichtung von Elektroden und Messung damit

Die Beschichtung der Elektroden erfolgt mit einem Siebdruckverfahren. Dazu wurde zunächst eine Paste hergestellt mit 15 Gew.% erfindungsgemäßem Konjugat (Ferrocen-HSA und Antikörper gegen HSA), 20 Gew.% Glucoseoxidase, 15 Gew.% Graphit, 1 Gew.% Glucose und 50 Gew.% Silikonöl.

Zur Herstellung der Paste liegt der Antikörper in wässriger Lösung vor und dazu wird die Verbindung aus Ferrocen und HSA sowie die Glucoseoxidase zugegeben und anschließende lyophilisiert, wodurch eine gute Durchmischung der Komponenten erreicht wird.

Die restlichen Komponenten der Paste werden anschließend zugegeben.

Das Mischen erfolgt manuell in an sich bekannter Weise unter Verwendung eines Dreiwalzenstuhls, wobei 5 bis 6 Mischzyklen durchgeführt wurden und die Temperatur der Walzen 40 bis 50°C nicht überschritt.

Die so erhaltene Paste wird in einem Lösungsmittel gemischt, wobei die Mischung 70 Gew.% Paste und 30 Gew.% Lösungsmittel erhielt. Das Lösungsmittel enthielt 90 % Cyclohexanon und 10 % Isophon.

Die Beschichtung der Elektroden mit der Paste erfolgte in einem konventionellen Siebdruckverfahren, wobei die Netze der Geometrie der Sensoren angepaßt wurden. Auf die verwendeten Kunststoffolien wurden Silberleitbahnen und Grafitflächen entsprechend der Sensorgeometrie aufgedruckt. Die Kapillaren wurden drucktechnisch aufgebaut.

Die Paste wurde mit dem Rakel gleichmäßig verteilt, wobei die Paste noch nicht durch die Löcher gequetscht wurde. Beim Drucken wurde das Sieb angeschlagen, drei Punkte links und rechts unten. Das Gewebe wurde auf die benötigte Breite mit wenig Druck gleichmäßig beschichtet. Dann wurde die Folie angehoben und mit dem Rakel bei einem Winkel von ca. 70-80 Grad und mit ca. 1-2 kg Druck gleichmäßig abgestriffen. Die Gewebeoberfläche wurde nun wieder von der Paste befreit. Zurück blieb nur noch eine hauchdünne Schicht, die lediglich eine gräuliche Färbung hinterließ. Der Anpressdruck der Folie konnte wieder gelöst werden.

Die Messung auf eventuell vorhandenes Antigen (HSA) erfolgt durch Verwendung eines üblichen Amperometers. Es wird bei einer Spannung von etwa 500 mV während etwa 30 Sekunden gemessen. Liegt in der zu untersuchenden Probe das Antigen vor, so wird ein Stromfluß gemessen, was sich in einem Peak bei der amperometrischen Messung darstellt. Wurde eine Antigen-haltige Probe verwendet, wurde ein solcher Peak beobachtet; wurde zur Messung eine Probe verwendet, die kein Antigen enthielt, konnte kein Stromfluß festgestellt werden.

## Patentansprüche

1. Konjugat zur Bestimmung von Antigenen mittels Biosensoren, das einen Mediator und ein Antigen, die kovalent miteinander verbunden sind, sowie einen gegen das Antigen gerichteten Antikörper, der über eine Antigen-Antikörper-Bindung gebunden ist, umfaßt.

2. Konjugat nach Anspruch 1, wobei der Mediator ein Ladungstransferkomplex, der Donor und Akzeptor aufweist, oder ein Redoxmediator ist.

3. Konjugat nach Anspruch 2, wobei der Redoxmediator Ferrocen, Nickelocen oder ein Derivat davon umfaßt.

4. Konjugat nach Anspruch 3, wobei das Ferrocen-Derivat Ferrocen-aminopropylpyrrol, Ferrocen-aminopentylamidopropylpyrrol oder eine Ferrocen-1,1'-C₁-C₁₀-Dicarbonsäure umfaßt.

5. Konjugat nach Anspruch 2, wobei der Donor ausgewählt ist unter Tetrathiofulvalen, Hexamethylentetraselenfulvalen, Tetramethyltetraselenfulvalen, N-Methylphenazon, Methylnorbornen-2,3-dicarbonsäureanhydrid, Chinolin, Kupfer-2,2-Dipyridylamin und 2,5-Dimethoxyzimtsäure.

6. Konjugat nach Anspruch 3, wobei der Akzeptor ausgewählt ist unter 7,7,8,8-Tetracyanochinodimethan, 7,7,8,8-Tetracyanochinodimethantetrafluorid, 2-[(3,5-Dinitro-phenyl)-(3-nitrophenyl)-methylen]-molononitril, 11,11,12,12-Teracyan-naphtochinodimethan(2,6) und Phenazinmethosulfat.

7. Konjugat nach Anspruch 3, wobei der Ladungstransferkomplex ausgewählt ist unter Tetrathiofulvalen-7,7,8,8-Tetracyanoquinodimethan, Mehyl-norbornen-2,3-dicarbonsäure-anhydrid-7,7,8,8-tetracyanoquinodimethan oder 1-Methyl-2-pyrrolidon-7,7,8,8-Tetracyanoquinodimethan

8. Konjugat nach einem der vorhergehenden Ansprüche, wobei das Antigen ausgewählt ist unter Proteine, Peptide, Glycoproteine, Lipoproteine und Nucleinsäuren.

9. Verfahren zum Nachweis eines Antigens mit einem Biosensor, der a) Elektroden, die eine elektrochemische Messung ermöglichen, b) das erfindungsgemäße Konjugat nach einem der Ansprüche 1-8, c) eine Oxidoreduktase mit Sauerstoff als Akzeptor und d) ein Substrat für die Oxidoreduktase aufweist, wobei der Biosensor mit der zu untersuchenden Probe in Kontakt und eine elektrochemische Messung durchgeführt wird.

10. Biosensor zum Bestimmen von Substanzen, wobei Elektroden vorgesehen sind, die zusammen mit einer enzymhaltigen Substanz eine elektrochemische Messung der zu bestimmenden Substanz erlauben, wobei der Sensor ein Konjugat nach einem der Ansprüche 1 bis 8 enthält.
